# FASCICULE DE BREVET EUROPEEN

(11) **EP 1 786 368 B1**
(45) Date de publication et mention de la délivrance du brevet: **20.08.2014**
(21) Numéro de dépôt: 05800585.1
(22) Date de dépôt: 07.09.2005
(51) Int. Cl.: A61F 2/24

(54) **PROTHESE VALVULAIRE**
VENTILPROTHESE
VALVE PROSTHESIS

(30) Priorité: 07.09.2004 FR 0409469
(43) Date de publication de la demande: 23.05.2007
(73) Titulaire: Laboratoires Perouse, 60173 Ivry Le Temple (FR)
(72) Inventeur: STYRC, Mikolaj, Witold, L-8190 Kopstal (LU)
(74) Mandataire: Blot, Philippe Robert Emile
(86) Numéro de dépôt international: PCT/FR2005/002228
(87) Numéro de publication internationale: WO 2006/027499

(56) Documents cités:
- FR-A- 2 847 800
- US-A- 4 680 031
- US-A- 4 790 843
- US-A1- 2003 125 793
- US-B2- 6 652 578

## Description

La présente invention concerne une prothèse valvulaire pour mise en place par voie endoluminale, du type comportant un obturateur souple et une armature porteuse annulaire qui est rigide radialement et propre à être implantée par voie chirurgicale à l'endroit d'une valve cardiaque.

Le coeur comporte deux oreillettes et deux ventricules qui sont séparés par des valves. Des valves sont en outre présentes en sortie du ventricule droit (valve pulmonaire) et du ventricule gauche (valve aortique).

Ces valves assurent une circulation univoque du flux sanguin, évitant un reflux sanguin à l'issue de la contraction ventriculaire.

Des maladies affectent les valves. En particulier, celles-ci peuvent souffrir d'une mauvaise ouverture, réduisant ainsi le flux sanguin, ou n'être que partiellement étanches, autorisant ainsi un reflux ou une régurgitation vers le ventricule ayant expulsé le flux sanguin.

Ces problèmes de régurgitation conduisent à une dilatation anormale du ventricule qui produit, à terme, une insuffisance cardiaque.

Il est connu de traiter ce type de maladie de manière chirurgicale, en remplaçant la valve malade. Les valves malades, et notamment la valve aortique en sortie du ventricule gauche sont remplacées par une valve prélevée sur un sujet décédé, ou par une valve prothétique couramment désignée par bioprothèse. Une valve prothétique est constituée d'une armature métallique annulaire et d'un obturateur souple réalisé dans un tissu d'origine animale. L'obturateur est solidarisé à demeure sur l'armature.

De telles valves sont décrites, notamment dans les documents WO-01/03095, WO-00/27975 et US 2003/0125 793.

Une fois implantée, l'armature s'applique contre la paroi intérieure du coeur à laquelle elle est cousue, notamment en entrée de l'artère aortique issue du ventricule gauche.

On constate, après plusieurs années d'implantation d'une telle prothèse, que celle-ci se dégrade et qu'elle ne fonctionne plus de manière efficace. En particulier, l'obturateur souple se déchire et présente des trous, ou l'obturateur se calcifie perdant ainsi sa souplesse et n'étant alors plus à même de se déformer pour exercer sa fonction de valve. Une nouvelle prothèse doit alors être mise en place.

Toutefois, le retrait de l'ancienne prothèse ne peut être effectué de manière endoluminale, notamment du fait que l'armature porteuse de la prothèse est cousue avec la paroi cardiaque, interdisant leur désolidarisation sans une intervention chirurgicale lourde prévoyant le remplacement complet de la valve.

Afin d'éviter une intervention chirurgicale lourde pour retirer l'ancienne prothèse et chercher à en fixer une seconde, il a été envisagé de disposer, par voie endoluminale, une nouvelle valve prothétique à l'intérieur de l'ancienne prothèse laissée en place.

La nouvelle valve prothétique est formée d'un support tubulaire en treillis déformable radialement équipé d'un obturateur souple disposé dans le conduit délimité par le support tubulaire. Cet obturateur est lié au support tubulaire et présente une forme telle qu'il soit propre, par déformation, à autoriser la circulation du sang dans un sens et à bloquer la circulation du sang, dans le sens opposé.

Il a été envisagé que le support tubulaire soit formé de fils métalliques élastiques entrelacés délimitant des mailles généralement en forme de losanges. Un tel support tubulaire est connu sous le terme de "stent". Ce support tubulaire est déformable entre une position de mise en place dans laquelle son diamètre est réduit et une position d'implantation dans laquelle son diamètre est plus important et est suffisant pour que le support s'appuie à l'intérieur de l'armature porteuse de l'ancienne prothèse.

Pour leur mise en place, de telles valves prothétiques comportant un support tubulaire en treillis sont disposées dans un cathéter de petit diamètre. L'extrémité du cathéter est amenée au travers du réseau artériel jusqu'à la région équipée de l'ancienne prothèse ne fonctionnant plus. La nouvelle valve prothétique est poussée hors du cathéter à l'aide d'un organe filiforme engagé dans le cathéter. Le support tubulaire étant élastique, celui-ci se déploie immédiatement et de manière autonome lorsqu'il n'est plus comprimé radialement par le cathéter. Il vient alors s'appliquer sur le pourtour intérieur de l'armature porteuse de l'ancienne prothèse.

La mise en place de la nouvelle prothèse et son déploiement sont très délicats surtout lorsque l'ancienne prothèse est très endommagée.

L'invention a pour but de proposer une prothèse valvulaire pouvant être remise en état facilement par voie endoluminale.

A cet effet, l'invention a pour objet une prothèse valvulaire, du type précité, caractérisée en ce qu'elle comporte :
- une valve prothétique interchangeable, indépendante de l'armature porteuse pour une mise en place par voie endoluminale au travers de ladite armature porteuse annulaire et comportant :
   . un support tubulaire déformable radialement par rapport à un axe principal entre, une position repliée de mise en place, et une position déployée d'implantation dans la structure porteuse, dans laquelle le support tubulaire s'applique à sa périphérie contre l'armature porteuse,
   . ledit obturateur souple lié au support tubulaire et déformable entre une position d'obstruction dans laquelle il est étendu transversalement et une position de libération dans laquelle il est contracté transversalement sous l'action du flux circulant au travers du support tubulaire,
en ce que l'armature porteuse forme un support annulaire dépourvu de tout obturateur propre à limiter de manière univoque la circulation du flux sanguin, en ce que le support tubulaire délimite une paroi cylindrique pleine, étanche aux liquides et en ce que le support tubulaire comporte un treillis tubulaire recouvert d'un film extensible et étanche aux liquides formant ladite paroi cylindrique pleine.

Suivant des modes particuliers de réalisation, la prothèse valvulaire comporte l'une ou plusieurs des caractéristiques suivantes :
- elle comporte au moins une membrure rigide s'étendant généralement suivant une génératrice du support tubulaire, laquelle membrure est liée au support tubulaire en au moins deux points espacés suivant l'axe du support tubulaire ;
- le support tubulaire présente un tronc médian généralement cylindrique et, axialement de part et d'autre du tronc, deux collerettes généralement tronconiques s'évasant depuis le tronc vers les extrémités du support ;
- le support tubulaire est élastique et est conformé pour être sollicité élastiquement de sa position repliée à sa position déployée ;
- la ou chaque membrure comporte une extrémité en saillie de liaison à un tuteur de maintien de la valve prothétique ;
- le support tubulaire est prolongé par des jambes convergentes formant trépied, lesquelles jambes sont liées entre elles en un point de liaison contenu sensiblement sur l'axe du support tubulaire ;
- l'obturateur comporte trois membranes déformables entre une position d'obturation dans laquelle les bords libres des membranes sont accolés deux à deux par moitié et une position de passage du flux sanguin dans laquelle les trois membranes sont écartées les unes des autres ;
- l'armature porteuse comporte une embase annulaire rigide et un ensemble de plots rigides s'étendant chacun depuis l'embase, parallèlement à l'axe de l'embase annulaire ; et
- l'armature porteuse comporte en surface, une nappe textile permettant la suture.

L'invention a, en outre pour objet, un nécessaire de traitement comportant :
- une prothèse valvulaire telle que décrite ci-dessus ; et
- un cathéter de mise en place de la valve prothétique.

L'invention sera mieux comprise à la lecture de la description qui va suivre, donnée uniquement à titre d'exemple et faite en se référant aux dessins, sur lesquels :
- la figure 1 est une vue en perspective de l'armature porteuse seule de la prothèse valvulaire qui est implantée de manière chirurgicale ;
- la figure 2 est une vue en perspective d'une prothèse valvulaire selon l'invention dans son état obturant;
- la figure 3 est une vue en bout de la prothèse valvulaire de la figure 2;
- la figure 4 est une vue identique à celle de la figure 3, la prothèse valvulaire étant dans son état passant ;
- les figures 5 et 6 sont des vues en coupe longitudinale illustrant les stades successifs de mise en place d'une valve prothétique d'une prothèse valvulaire selon l'invention ;
- les figures 7 et 8 sont des vues identiques à celles des figures 5 et 6 illustrant des stades successifs de retrait d'une valve prothétique d'une prothèse valvulaire selon l'invention.

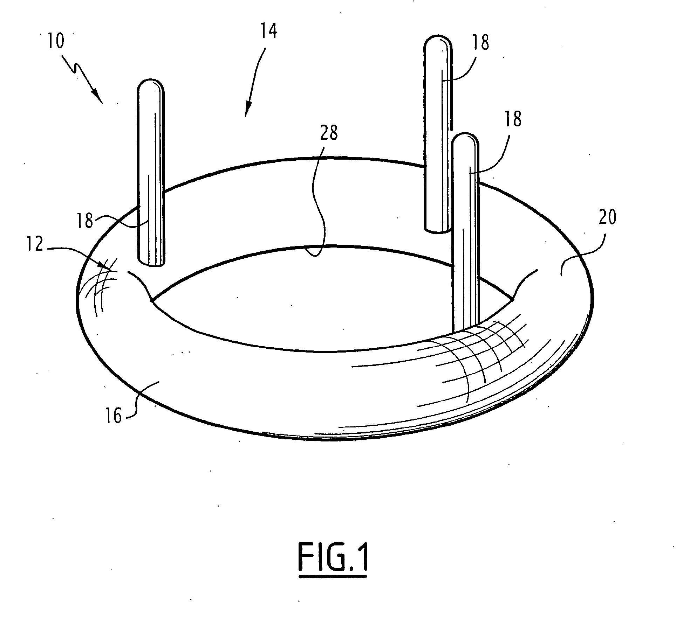

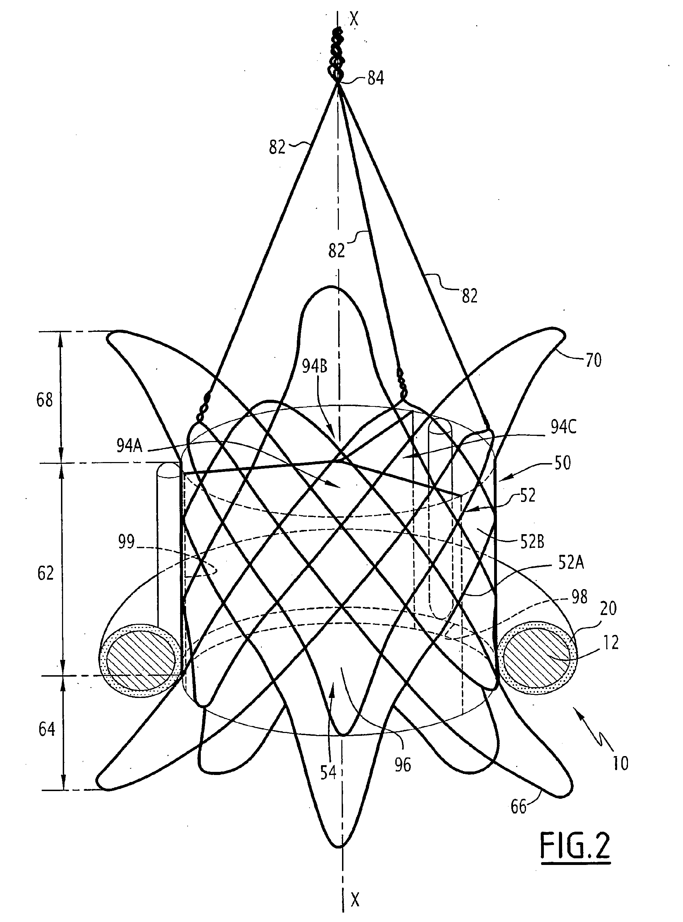

Sur les figures 1 et 2 est représentée une prothèse valvulaire 10 qui est visible au complet sur la figure 2 et seulement partiellement sur la figure 1. La prothèse valvulaire est destinée à une valve aortique du coeur. Ainsi, cette prothèse est placée immédiatement en amont de l'aorte à l'emplacement de la valve naturelle.

La prothèse comporte une armature porteuse 12 visible seule sur la figure 1. Cette armature comporte essentiellement une embase annulaire rigide 16 portant trois plots 18 rigides s'étendant chacun depuis l'embase parallèlement à l'axe de l'embase annulaire 16. Cette embase est formée d'un tore métallique rigide auquel sont soudés les trois plots 18. Le tore est enveloppé sur toute sa surface d'une nappe textile tissée 20 permettant la solidarisation de l'armature porteuse au tissu organique du coeur par suture entre la nappe textile et la paroi organique du coeur. Le diamètre intérieur de l'embase annulaire 16 est compris entre 15 mm et 40 mm.

Les plots 18 sont liés, depuis une extrémité, à l'embase annulaire 16 et font tous saillie du même côté. Ils sont régulièrement répartis angulairement autour de l'axe de l'armature porteuse 12. La hauteur totale des plots 18 y compris l'embase annulaire 16 est comprise entre 10 mm et 30 mm.

L'armature porteuse 12 est dépourvue de tout obturateur souple déformable dans l'espace définie par l'armature entre une position d'obturation et une position de passage.

Sur la figure 2 est représentée une prothèse valvulaire 10 selon l'invention après implantation. La prothèse valvulaire comporte en plus de l'armature porteuse 12 une valve prothétique 50 interchangeable par voie endoluminale. A l'état implanté, la valve prothétique s'étend à l'intérieur de l'armature porteuse 12 préalablement implantée par voie chirurgicale.

La valve prothétique 50 comporte un support tubulaire en treillis 52 d'axe X-X et un obturateur souple 54 lié au support tubulaire 52 et disposé à l'intérieur de celui-ci.

La valve 50 est remplaçable et est notamment amovible par rapport à l'armature porteuse 12.

Le support tubulaire 52 est constitué, par exemple, d'un treillis tubulaire 52A noyé dans un film 52B extensible et étanche aux liquides tel qu'un élastomère. Le film 52B recouvrant le treillis, définit suivant toute la hauteur du support 52 une paroi cylindrique pleine, étanche aux liquides. Le treillis 52A est constitué d'acier inoxydable ayant des propriétés élastiques, de sorte que le support 52 est auto-expansible. Un tel support lorsqu'il est utilisé seul est couramment désigné par le terme anglais "stent".

Comme connu en soi, le support 52 est susceptible de se déformer spontanément d'un état comprimé, dans lequel il présente un petit diamètre à un état dilaté, dans lequel il présente un diamètre supérieur, cet état dilaté constituant son état de repos.

Dans son état implanté, tel qu'illustré sur les figures 2 à 4, le support 52 s'applique, du fait de son élasticité, contre l'embase annulaire 16 et les plots 18 de l'armature porteuse, en maintenant les trois membranes 26 appliquées sur la surface externe du support 52.

A chacune de ses extrémités axiales, le support 52 se prolonge axialement au-delà de l'armature porteuse par deux collerettes divergentes présentant une forme généralement tronconique s'évasant vers les extrémités axiales du support.

Plus précisément, le support 52 présente un tronc médian 62 généralement cylindrique dont la longueur correspond à la hauteur de l'armature porteuse, cette hauteur étant mesurée suivant l'axe de la valve. La hauteur du tronc est comprise entre 10 mm et 30 mm.

Le treillis délimitant le tronc 62 est formé de fils métalliques entrelacés. Ainsi, deux familles de fils s'entrecroisent. Les fils de la première famille décrivent des hélices orientées dans un même sens et s'étendant généralement parallèlement les unes aux autres. Au contraire, les fils de la seconde famille décrivent des hélices orientées en sens inverse et s'étendant parallèlement les unes aux autres. Les fils des première et seconde familles sont engagés successivement au-dessus et au-dessous les uns des autres, de sorte que ces familles de fils délimitent des mailles en forme de losanges, dont une diagonale de chaque maille s'étend suivant l'axe du support, et dont l'autre diagonale s'étend généralement perpendiculairement.

A une première extrémité du support, le tronc 62 est prolongé par une collerette évasée 64 formée d'un ensemble de lobes 66 s'écartant de l'axe du support en direction de leur extrémité recourbée. Ces lobes sont formés de boucles formées aux extrémités des fils des première et seconde familles, lesquels sont venus de matière.

De même, à son autre extrémité, le support comporte une seconde collerette évasée 68 prolongeant le tronc 62. Celle-ci est également délimitée par des lobes 70 déformés vers l'extérieur.

Au repos, l'extrémité libre des collerettes, c'est-à-dire la section extrême la plus évasée de ces collerettes, définit un contour dont le diamètre est égal au diamètre du tronc 62 augmenté de 5 mm à 15 mm.

De même, avantageusement, la hauteur des collerettes 64, 68 mesurée suivant l'axe du support tubulaire 52 est comprise entre 5 mm et 15 mm et est par exemple égale à 10 mm.

Le film 52B dans lequel est noyé le treillis tubulaire 52A se prolonge sur les lobes formant les collerettes 64 et 68.

Suivant un premier mode de réalisation, le support tubulaire 52 présente sur toute sa hauteur au repos, lorsqu'il n'est pas comprimé dans une armature 12, un diamètre supérieur au diamètre de l'armature 12, de sorte que les collerettes 64 et 68 se mettent en forme évasée du seul fait de l'élasticité naturelle du support tubulaire alors que le tronc est maintenu confiné sous forme tubulaire dans l'armature porteuse 12.

En variante, le tronc 62 du support tubulaire présente, au repos, même lorsqu'il n'est pas comprimé dans une armature 12, un diamètre inférieur au diamètre d'extrémité des collerettes 64 et 68.

Par ailleurs, trois paires de fils issus des première et seconde familles respectivement se relient par paire l'un à l'autre au niveau de la collerette 68 pour former trois jambes 82. Les jambes convergent l'une vers l'autre suivant l'axe X-X de la valve prothétique pour se réunir en un point de liaison 84 contenu sur cet axe. Les trois jambes 82 délimitent ainsi un trépied. Elles sont angulairement régulièrement réparties autour de l'axe X-X et délimitent chacune avec cet axe un angle compris entre 20° et 40°. Pour leur liaison, les trois jambes 82 sont par exemple torsadées ensemble au point 34. Une boucle d'accrochage est formée au point extrême 34.

En outre, et avantageusement, le support tubulaire 52 comporte au moins une membrure rigide 90 s'étendant généralement suivant une génératrice du support tubulaire 52. Cette membrure est liée au support en au moins deux points 92A, 92B espacés suivant l'axe du support. Ces deux points sont formés sur la hauteur du tronc 62, notamment au voisinage de la région de liaison avec les collerettes 64 et 68. La liaison est effectuée par soudage ou collage.

Avantageusement, une unique membrure 90 est formée suivant une génératrice du tronc 62. Cette membrure est constituée par exemple d'un fil métallique rigide longitudinalement qui est engagé au travers des mailles du treillis alternativement à l'intérieur et à l'extérieur du treillis.

Avantageusement, les extrémités de la membrure sont disposées à l'intérieur du support tubulaire, c'est-à-dire du côté de l'axe X-X par rapport au film étanche 52B.

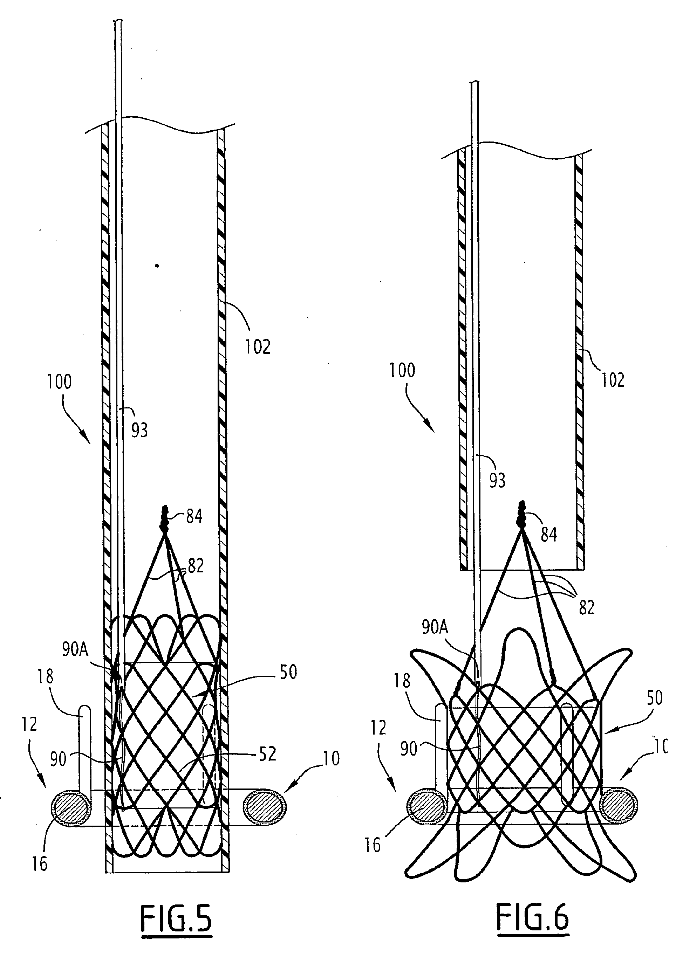

Une extrémité en saillie 90A de la membrure 90 au moins, et notamment celle du côté des jambes 82 est propre à coopérer avec un tuteur 93 pour assurer leur solidarisation axiale, comme illustré sur la figure 5 et comme cela sera expliqué ultérieurement. La solidarisation axiale entre le tuteur 93 et la membrure 90 est assurée, par exemple, par emboîtement de l'extrémité de liaison 90A de la membrure dans un logement prévu dans l'épaisseur du tuteur 93 et débouchant au bout de celui-ci.

L'obturateur 54 est relié à la surface intérieure du support tubulaire 52. Cet obturateur est formé de trois membranes souples 94A, 94B, 94C, chacune formée d'un film de polymère ou d'une couche de film organique telle que du péricarde de veau. Chaque membrane est généralement de forme rectangulaire. Elle est liée à la surface interne du film étanche 52B suivant un grand côté 98 formant base suivant la circonférence de liaison entre le tronc 62 et la collerette élargie 64.

Les bords longitudinaux 99 des trois membranes 94A, 94B, 94C, sont liés au support tubulaire 52 suivant trois génératrices de celui-ci réparties régulièrement de manière angulaire autour de l'axe du support tubulaire. Ainsi, les membranes sont liées deux à deux suivant leurs bords longitudinaux sur le support tubulaire. Cette liaison s'effectue sur toute la hauteur du tronc 62.

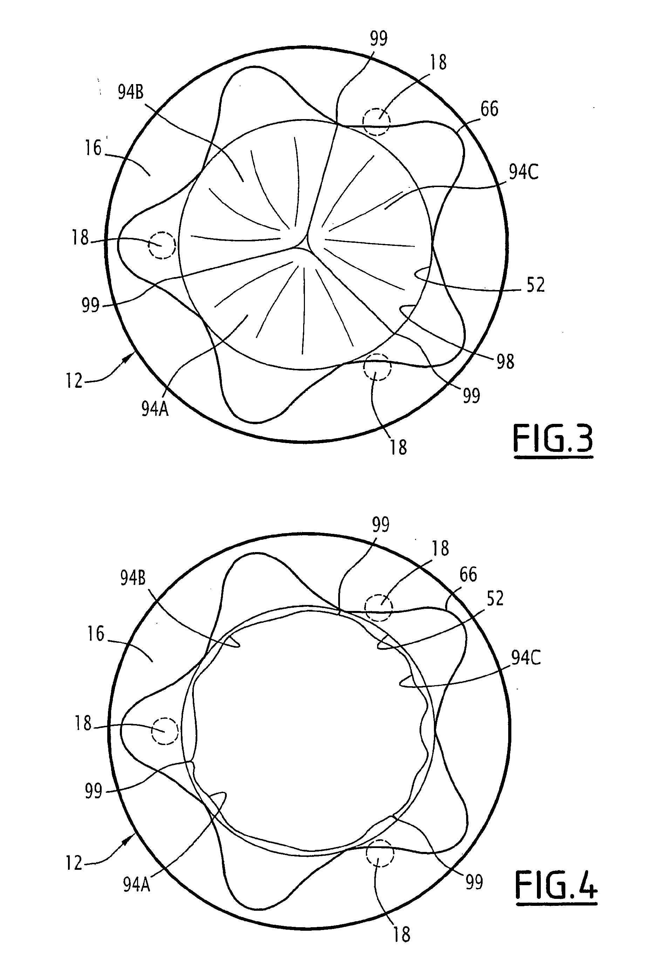

Les membranes 94A, 94B, 94C formant l'obturateur sont déformables entre une position d'obturation représentée sur les figures 2 et 3 dans laquelle les bords libres des membranes sont accolées deux à deux par moitié et une position de passage du flux sanguin illustrée sur la figure 4 dans laquelle les trois membranes sont écartées les unes des autres.

Dans la position d'obturation, les trois membranes délimitent, avec la paroi tubulaire du support 52, trois poches de retenue du flux sanguin. Au contraire, dans la position de passage, les trois membranes sont appliquées sur la surface intérieure du support tubulaire, comme illustré sur la figure 4, délimitant ainsi ensemble un conduit généralement circulaire pour la circulation du flux sanguin.

Pour la mise en place initiale de la prothèse valvulaire, le chirurgien procède d'abord à la mise en place par voie chirurgicale de l'armature porteuse 12. A cet effet, une incision est pratiquée dans le thorax du patient pour acheminer l'armature porteuse 12 jusqu'au coeur où celle-ci est implantée en lieu et place de la valve originale. L'armature porteuse 12 est liée à la paroi du coeur par des fils de suture engagés dans le revêtement textile 20 de l'embase annulaire.

Lors de l'implantation initiale de la prothèse valvulaire, la valve prothétique 50 est mise en place manuellement à l'intérieur de l'armature porteuse 12, après quoi le thorax du patient est recousu.

L'armature 12 est implantée définitivement dans le corps du patient alors que la valve prothétique 50 est interchangeable. Ainsi, lorsque la valve prothétique 50 est endommagée, notamment parce que les membranes sont calcifiées ou sont déchirées, la valve prothétique est extraite par voie endoluminale comme cela sera exposé dans la suite et une nouvelle valve prothétique est mise en place dans l'espace délimité par l'armature porteuse 12 comme exposé ci-dessous.

Pour la mise en place par voie endoluminale d'une nouvelle valve prothétique 50, un nécessaire de traitement 100 illustré sur les figures 5 et 6 est mis en oeuvre. Il comporte une nouvelle valve prothétique 50 contenue dans un cathéter 102 de diamètre extérieur inférieur au diamètre intérieur de l'armature porteuse 12.

Comme illustré sur la figure 5, la valve prothétique, et notamment le support tubulaire 52, est comprimé radialement à l'intérieur du tube.

En outre, le tuteur 93 s'étend suivant la longueur du cathéter 102 en étant solidarisé depuis son extrémité à l'extrémité de la membrure de rigidification axiale 90. Le tuteur 93 présente axialement une rigidité suffisante pour pousser la valve prothétique hors du cathéter 102.

Pour la mise en place, l'extrémité du cathéter 102 dans lequel est logée la valve prothétique est introduite dans l'aorte du patient puis est déplacée progressivement dans l'aorte jusqu'à l'emplacement de l'armature porteuse 12 en sortie du coeur. Le déplacement du cathéter s'effectue alors à contre-courant du flux sanguin normal.

Le cathéter est amené dans la position illustrée sur la figure 5. Dans cette position, le cathéter 102 est alors tiré alors que la nouvelle valve prothétique 50 est maintenue en place par le tuteur 93. Au fur et à mesure du déplacement du cathéter 102, la valve prothétique 50 se trouve découverte, de sorte que son extrémité se déploie pour former la collerette 64 puis le tronc 62 de support tubulaire vient s'appliquer contre les plots 18 et enfin son autre extrémité se déploie pour former la collerette 68.

Lors de la mise à nu progressive de la valve prothétique 50 par déplacement du cathéter 102, la valve prothétique est maintenue fixe axialement par rapport au conduit aortique et notamment par rapport à l'armature porteuse 12 laissée en place grâce au tuteur rigide 93 qui retient dans son prolongement la membrure 90. Ainsi, la présence du tuteur 93 coopérant avec la membrure 90 réduit les risques de déplacement axial de la valve prothétique pendant son largage, même si celle-ci est larguée lors d'une pulsation cardiaque conduisant à une mise en circulation du sang à l'endroit d'implantation de la valve.

Après déploiement, la valve est maintenue axialement par la présence des collerettes élargies 64 et 68 s'appuyant respectivement sur l'embase annulaire 16 et l'extrémité des plots 18.

Après largage, le tuteur 93 est retiré par simple traction. Ainsi, la membrure 90 se désemboîte de l'extrémité du tuteur 93. La membrure 90 reste en place puisqu'elle est intégrée au support tubulaire 52.

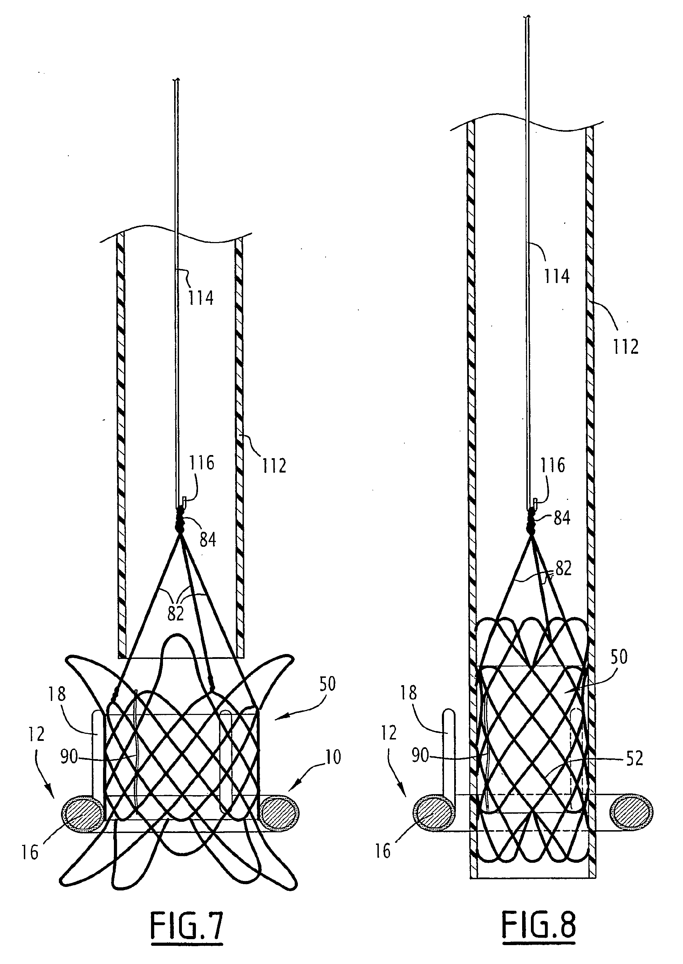

Comme illustré sur les figures 7 et 8, pour le retrait d'une valve prothétique endommagée 50, un cathéter 112 est introduit au travers de l'aorte et est disposé en regard de l'extrémité de la valve présentant le trépied formé des jambes 82.

Un outil de traction 114 est acheminé au travers du cathéter 112. Cet outil comporte, à son extrémité, une crosse 116 permettant d'accrocher le point de liaison 84 du trépied. Alors que l'extrémité ouverte du cathéter est en contact avec les jambes 82 du trépied, la valve prothétique 50 est progressivement introduite à l'intérieur du cathéter 112 en avançant le cathéter 112 suivant la longueur de la valve 50. Par effet de came, les jambes 82 sont resserrées vers l'axe et la valve prothétique est progressivement amenée dans son état resserré et introduite dans le cathéter 112, comme illustré sur la figure 8. Le cathéter 112 renfermant la valve prothétique 50 est alors extrait du corps humain.

Une nouvelle valve prothétique 50 est alors introduite à l'aide d'un nécessaire de traitement 100 dans le corps humain et la valve est larguée comme exposé précédemment.

On comprend qu'avec une telle prothèse vasculaire, une unique intervention chirurgicale lourde est nécessaire pour la mise en place de l'armature porteuse 12, puis que la valve prothétique peut être changée périodiquement par voie endoluminale, ce qui est une intervention légère pour le patient.

L'absence de tout élément formant obturateur sur l'armature porteuse qui a pour seule fonction de constituer un appui rigide pour la valve prothétique permet d'avoir une surface d'appui satisfaisante et propre quel que soit l'état de la valve prothétique.

Au contraire, lorsqu'une valve prothétique formée d'un stent équipé d'un obturateur est implantée au travers d'une valve prothétique endommagée comportant une armature porteuse et un obturateur, la présence de l'obturateur, souvent calcifié gêne la mise en place de la nouvelle valve prothétique.

## Revendications

1. Prothèse valvulaire (10) comportant :
- un obturateur souple (54) et une armature porteuse annulaire (12) qui est rigide radialement et propre à être implantée par voie chirurgicale à l'endroit d'une valve cardiaque,
**caractérisée en ce qu'**elle comporte :
- une valve prothétique (50) interchangeable, indépendante de l'armature porteuse (12) pour une mise en place par voie endoluminale au travers de ladite armature porteuse annulaire (12) et comportant :
. un support tubulaire (52) déformable radialement par rapport à un axe principal (X-X) entre, une position repliée de mise en place, et une position déployée d'implantation dans la structure porteuse, dans laquelle le support tubulaire (52) s'applique à sa périphérie contre l'armature porteuse (12),
. ledit obturateur souple (54) lié au support tubulaire (52) et déformable entre une position d'obstruction dans laquelle il est étendu transversalement et une position de libération dans laquelle il est contracté transversalement sous l'action du flux circulant au travers du support tubulaire (52),
**en ce que** l'armature porteuse (12) forme un support annulaire dépourvu de tout obturateur propre à limiter de manière univoque la circulation du flux sanguin,
**en ce que** le support tubulaire (52) délimite une paroi cylindrique pleine, étanche aux liquides,
et **en ce que** le support tubulaire (52) comporte un treillis tubulaire (52A) recouvert d'un film (52B) extensible et étanche aux liquides formant ladite paroi cylindrique pleine.

2. Prothèse valvulaire (10) selon la revendication 1, **caractérisée en ce qu'**elle comporte au moins une membrure rigide (90) s'étendant généralement suivant une génératrice du support tubulaire (52), laquelle membrure (90) est liée au support tubulaire (52) en au moins deux points (92A, 92B) espacés suivant l'axe du support tubulaire (52).

3. Prothèse valvulaire (50) selon la revendication 2, **caractérisée en ce que** la ou chaque membrure (90) comporte une extrémité en saillie (90A) de liaison à un tuteur (93) de maintien de la valve prothétique (10).

4. Prothèse valvulaire (10) selon l'une quelconque des revendications précédentes, **caractérisée en ce que** le support tubulaire (52) présente un tronc médian (62) généralement cylindrique et, axialement de part et d'autre du tronc (62), deux collerettes (66, 70) généralement tronconiques s'évasant depuis le tronc (62) vers les extrémités du support (52).

5. Prothèse valvulaire (10) selon l'une quelconque des revendications précédentes, **caractérisée en ce que** le support tubulaire (52) est élastique et est conformé pour être sollicité élastiquement de sa position repliée à sa position déployée.

6. Prothèse valvulaire (10) selon l'une quelconque des revendications précédentes, **caractérisée en ce que** le support tubulaire (52) est prolongé par des jambes convergentes (82) formant trépied, lesquelles jambes sont liées entre elles en un point de liaison (84) contenu sensiblement sur l'axe (X-X) du support tubulaire (52).

7. Prothèse valvulaire (10) selon l'une quelconque des revendications précédentes, **caractérisée en ce que** l'obturateur (50) comporte trois membranes (94A, 94B, 9AC) déformables entre une position d'obturation dans laquelle les bords libres des membranes sont accolés deux à deux par moitié et une position de passage du flux sanguin dans laquelle les trois membranes sont écartées les unes des autres.

8. Prothèse valvulaire (10) selon l'une quelconque des revendications précédentes, **caractérisée en ce que** l'armature porteuse (12) comporte une embase annulaire rigide (16) et un ensemble de plots rigides (18) s'étendant chacun depuis l'embase (16), parallèlement à l'axe de l'embase annulaire (16).

9. Prothèse valvulaire (10) selon l'une quelconque des revendications précédentes, **caractérisée en ce que** l'armature porteuse comporte en surface, une nappe textile (20) permettant la suture.

10. Nécessaire de traitement (100) comportant :
. une prothèse valvulaire (12,50) selon l'une quelconque des revendications précédentes ; et
. un cathéter (102) de mise en place de la valve prothétique (50).

## Patentansprüche

1. Klappenprothese (10), aufweisend:
- ein flexibles Verschlussstück (54) und eine ringförmige Trägerarmatur (12), die radial steif ist und geeignet ist, auf chirurgischem Weg an die Stelle einer Herzklappe implantiert zu werden,
**dadurch gekennzeichnet, dass** sie aufweist:
- eine austauschbare prothetische Klappe (50), die von der Trägerarmatur (12) unabhängig ist, für ein Einsetzen auf endoluminalem Weg durch die ringförmige Trägerarmatur (12) hindurch, und die aufweist:
• eine rohrförmige Stütze (52), die bezüglich einer Hauptachse (X-X) radial verformbar ist zwischen einer Zusammenklapp-Position zum Einsetzen und einer Entfaltungsposition zum Implantieren in der Trägerstruktur, in der die rohrförmige Stütze (52) an ihrem Umfang gegen die Trägerarmatur (12) drückt,
• wobei das flexible Verschlussstück (54) mit der rohrförmigen Stütze (52) verbunden ist und verformbar ist zwischen einer Verschlussposition, in der es transversal ausgebreitet ist, und einer Freigabeposition, in der es unter der Wirkung des durch die rohrförmige Stütze (52) hindurchzirkulierenden Stroms transversal zusammengezogen ist,
dass die Trägerarmatur (12) eine ringförmige Stütze ohne Verschlussstück ausbildet, das geeignet ist, das Zirkulieren des Blutstroms auf eine Richtung zu begrenzen,
dass die rohrförmige Stütze (52) eine solide zylinderförmige Wand begrenzt, die gegenüber Flüssigkeiten dicht ist,
und dass die rohrförmige Stütze (52) ein rohrförmiges Gitter (52A) aufweist, das von einem dehnbaren und gegenüber Flüssigkeiten dichten Film (52B) bedeckt ist, der die solide zylinderförmige Wand ausbildet.

2. Klappenprothese (10) gemäß Anspruch 1, **dadurch gekennzeichnet, dass** sie mindestens eine steife Rippe (90) aufweist, die sich im Wesentlichen entlang einer Mantellinie der rohrförmigen Stütze (52) erstreckt, wobei die Rippe (90) an mindestens zwei Punkten (92A, 92B), die entlang der Achse der rohrförmigen Stütze (52) im Abstand angeordnet sind, mit der rohrförmigen Stütze (52) verbunden ist.

3. Klappenprothese (50) gemäß Anspruch 2, **dadurch gekennzeichnet, dass** die oder jede Rippe (90) ein hervorstehendes Ende (90A) zum Verbinden mit einer Stütze (93) zum Halten der prothetischen Klappe (10) aufweist.

4. Klappenprothese (10) gemäß irgendeinem der vorhergehenden Ansprüche, **dadurch gekennzeichnet, dass** die rohrförmige Stütze (52) einen im Wesentlichen zylinderförmigen medianen Kegelstumpf (62) und axial auf beiden Seiten des Kegelstumpfes (62) zwei im Wesentlichen kegelstumpfförmige Flansche (66, 70) aufweist, die von dem Kegelstumpf (62) aus in Richtung zu den Enden der Stütze (52) weiter werden.

5. Klappenprothese (10) gemäß irgendeinem der vorhergehenden Ansprüche, **dadurch gekennzeichnet, dass** die rohrförmige Stütze (52) elastisch ist und angepasst ist, um aus ihrer Zusammenklapp-Position in ihre Entfaltungsposition federbelastet zu werden.

6. Klappenprothese (10) gemäß irgendeinem der vorhergehenden Ansprüche, **dadurch gekennzeichnet, dass** die rohrförmige Stütze (52) durch konvergente Schenkel (82), die ein Dreibein ausbilden, verlängert ist, wobei die Schenkel an einem Verbindungspunkt (84), der im Wesentlichen auf der Achse (X-X) der rohrförmigen Stütze (52) enthalten ist, miteinander verbunden sind.

7. Klappenprothese (10) gemäß irgendeinem der vorhergehenden Ansprüche, **dadurch gekennzeichnet, dass** das Verschlussstück (50) drei Membranen (94A, 94B, 9AC) aufweist, die zwischen einer Verschlussposition, in der die freien Ränder der Membranen paarweise zur Hälfte aneinandergefügt sind, und einer Position zum Durchlassen des Blutflusses, in der die drei Membranen voneinander entfernt sind, verformbar sind.

8. Klappenprothese (10) gemäß irgendeinem der vorhergehenden Ansprüche, **dadurch gekennzeichnet, dass** die Trägerarmatur (12) einen steifen ringförmigen Sockel (16) und einen Satz steifer Stifte (18) aufweist, die sich jeweils von dem Sockel (16) aus parallel zu der Achse des ringförmigen Sockels (16) erstrecken.

9. Klappenprothese (10) gemäß irgendeinem der vorhergehenden Ansprüche, **dadurch gekennzeichnet, dass** die Trägerarmatur an der Oberfläche eine Textillage (20) aufweist, die das Nähen ermöglicht.

10. Behandlungskit (100), aufweisend:
• eine Klappenprothese (12, 50) gemäß irgendeinem der vorhergehenden Ansprüche, und
• einen Katheter (102) zum Einsetzen der prothetischen Klappe (50).

## Claims

1. A valve prosthesis (10) comprising:
· a flexible shutter (54) and an annular carrier structure (12) that is radially rigid and suitable for being surgically-implanted at the location of a heart valve;
the prosthesis being **characterized in that** it further comprises:
· an interchangeable prosthetic valve (50) independent of the carrier structure (12) for putting into place by an endoluminal approach through said annular carrier structure (12) and comprising:
· a tubular support (52) that is radially deformable relative to a main axis (X-X) between a folded position for putting into place, and a deployed position implanted in the carrier structure, in which the tubular support (52) bears at its periphery against the carrier structure (12);
· said flexible shutter (54) being connected to the tubular support (52) and deformable between an obstruction position in which it is extended transversally and a release position in which it is contracted transversally under the action of the blood flowing through the tubular support (52);
· **in that** the carrier structure (12) forms an annular support having no shutter suitable for restricting the flow of blood to one direction only;
· **in that** the tubular support (52) defines a solid cylindrical wall that is liquid proof; and
· **in that** the tubular support (52) comprises a tubular lattice (52A) covered in a stretchable film (52B) that is liquid-proof and that forms said solid cylindrical wall.

2. A valve prosthesis (10) according to claim 1, **characterized in that** it includes at least one rigid member (90) extending generally along a generator line of the tubular support (52), which member (90) is connected to the tubular support (52) at at least two points (92A, 92B) that are spaced apart along the axis of the tubular support(52).

3. A valve prosthesis (50) according to claim 2, **characterized in that** the or each member (90) has a projecting end (90A) for connection to a prop (93) for holding the prosthetic valve (10) in position.

4. A valve prosthesis (10) according to any preceding claim, **characterized in that** the tubular support (52) presents a generally cylindrical middle trunk (62) and, axially at either end of the trunk (62), two generally frustoconical collars (66, 70) flaring from the trunk (62) towards the ends of the support (52).

5. A valve prosthesis (10) according to any preceding claim, **characterized in that** the tubular support (52) is resilient and is shaped to be urged resiliently from its folded position towards its deployed position.

6. A valve prosthesis (10) according to any preceding claim, **characterized in that** the tubular support (50) is extended by converging legs (82) forming a tripod, which legs are connected together at a connection point (84) lying substantially on the axis (X-X) of the tubular support (52).

7. A valve prosthesis (10) according to any preceding claim, **characterized in that** the shutter (50) comprises three membranes (94A, 94B, 94C) that are deformable between a closed position in which the free edges of the membranes, over half their length, touch one another in pairs, and an open position for passing blood in which the three membranes are spaced apart from one another.

8. A valve prosthesis (10) according to any preceding claim, **characterized in that** the carrier structure (12) comprises a rigid ring (16) and a set of rigid pegs (18) each extending from the ring (16) parallel to the axis of the ring (16).

9. A valve prosthesis (10) according to any preceding claim, **characterized in that** the carrier structure includes at its surface a textile sheet (20) making suturing possible.

10. A treatment kit 100) comprising:
· a valve prosthesis (12,50) according to any preceding claim; and
· a catheter (102) for putting the prosthetic valve (50) into place.
